# EUROPEAN PATENT APPLICATION

(11) **EP 4 706 702 A1**
(43) Date of publication of application: **11.03.2026**
(21) Application number: 25200543.4
(22) Date of filing: 05.09.2025
(51) Int. Cl.: A61M 1/16, A61M 16/06

(54) **RECYCLING OXYGEN DURING ECMO THERAPY FOR NASAL CANNULATION**

(30) Priority: 06.09.2024 US 202463691376 P
(71) Applicant: Breethe, Inc., Halethorpe, MD 21227 (US)
(72) Inventor: COURNANE, Stephen, Halethorpe, 21227 (US); ZHANG, Jiafeng, Halethorpe, 21227 (US)
(74) Representative: ABG Intellectual Property Law, S.L.

(57) **Abstract**

An extracorporeal membrane oxygenation (ECMO) system for infusing blood with oxygen and for removing carbon dioxide from blood includes an outlet from the oxygen line to share oxygen from the oxygen source as between the system and a nasal cannula or other supplemental oxygen device. The ECMO system may alternatively or in addition capture oxygen-rich exhaust as a by-product of the process. This captured oxygen-rich exhaust would be recycled and shared with a nasal cannula or other supplemental oxygen device. The recycled oxygen-rich exhaust is not only richer in oxygen than air, but also warm and moist which may benefit patients beyond cold and dry oxygen from a direct oxygen source.

## Description

### CROSS-REFERENCE TO RELATED APPLICATIONS

This application claims the benefit of the filing date of United States Provisional Patent Application No. 63/691,376 filed September 6, 2024, the disclosure of which is hereby incorporated herein by reference.

### BACKGROUND

The present disclosure relates to the recovery of a patient undergoing extracorporeal membrane oxygenation (ECMO), sometimes also referred to as extracorporeal life support (ECLS), and more particularly to a system for sharing oxygen from extracorporeal oxygenation for other use by a recovering patient.

Patients awaiting or recovering from a heart or lung transplant and patients having conditions putting them at risk for heart or lung failure may be candidates for ECMO therapy. ECMO therapy is designed to address an excess of carbon dioxide and/or lack of oxygen in the blood where the lungs are not healthy and/or the heart cannot pump enough blood around the body. ECMO is thus an extracorporeal technique which replaces these respiratory and cardiac functions in various situations ranging from support needed while separately treating the underlying causes of cardiac arrest to late-stage treatment for heart or lung failure. It may be a therapeutic treatment to provide temporary help when needed.

ECMO therapy operates by drawing deoxygenated blood from the body of a patient in a controlled manner to permit oxygenation of the blood and to remove carbon dioxide from the blood. The oxygenated blood is then cycled back into the patient. Generally, ECMO systems include a pump, an oxygenator, an oxygen source and a control center to monitor and control the process. It may also include a blood warmer to bring the blood back up to the temperature of the body as it is circulated back into the body. Deoxygenated blood is drawn from the body via a cannula is pumped through an oxygenator where oxygen is infused in the blood and carbon dioxide is removed from the blood as a result of such infusion. The oxygenated blood is pumped from the oxygenator back into the body, with blood warming in most instances.

### BRIEF SUMMARY OF THE INVENTION

In accordance with a first aspect of the present disclosure, a blood oxygenator includes a housing having an oxygenator fiber bundle, a blood inlet to the bundle, a blood outlet, an oxygen inlet to the bundle for oxygenating the blood of a patient and an oxygen outlet, as well as an oxygen source to provide oxygen to the oxygen inlet, a pump for pumping oxygen through an oxygen feed conduit to the fiber bundle and a supplemental oxygen device to administer oxygen to the patient separately from the oxygenation of the blood. A portion of oxygen from the oxygen source is provided to the supplemental oxygen device.

In another aspect of the present disclosure, the oxygenator may also include a collection device to capture gas discharged from the oxygenator at the oxygen outlet to provide the oxygen to the supplemental oxygen device.

In yet another aspect of the present disclosure, a supplemental oxygen device may be a nasal cannula. An air filter may be provided and may be located after the oxygen outlet. An oxygen pump may be provided and may also be located after the oxygen outlet, and may also be situated downstream from the air filter if there is an air filter. An oxygen pressure regulator may be provided and may be located after the oxygen outlet. Various permutations of these aspects are contemplated whereby the features may be combined in various forms. By way of example, this aspect may include an air filter and an oxygen pump, it may include an air filter and pressure regulator, or it may include an air filter, oxygen pump and a pressure regulator.

In yet another aspect of the present disclosure, a diverter may be provided upstream of the oxygen inlet so as to provide oxygen to the supplemental oxygen device. In this aspect, the alternate and combined aspects in the foregoing paragraph may also be provided. In addition, this aspect may also have a humidifying device or a warming and humidifying device and it may be located after the oxygen outlet.

In yet a further aspect of the present disclosure, a method of administering oxygen to a patient includes the steps of providing a housing having within it a fiber bundle and a blood inlet, a blood outlet, an oxygen inlet and an oxygen outlet, directing blood from a patient through the blood inlet into the fiber bundle, pumping oxygen through an oxygen feed conduit extending from an oxygen source to the oxygen inlet, whereby an amount of oxygen is imparted into the fiber bundle to infuse the blood with oxygen, and directing a portion of the oxygen pumped to the oxygen inlet for supplemental oxygen administration to the patient separately from the oxygenation of the blood.

In yet a further aspect of the present disclosure, the above method may include the step of diverting oxygen from the oxygen feed conduit to a shared conduit to a supplemental oxygen device. An alternative aspect may include the step of capturing at the oxygen outlet a discharge gas imparted into the oxygen conduit from the fiber bundle and the step of directing the captured gas for supplemental oxygen administration to the patient separately from the oxygenation of the blood.

In any of the foregoing aspects of the method, there may be a step of filtering the oxygen for supplemental oxygen administration. A step of humidifying, or warming and humidifying, the oxygen for administration may be introduced. A step of regulating the pressure of the oxygen is also contemplated as an optional step. A step of removing excess moisture may also be implemented.

### BRIEF DESCRIPTION OF THE DRAWINGS

The drawings described herein are for illustrative purposes only of the selected embodiments and are not all possible implementations and thus are not intended to limit the scope of the present disclosure.
FIG. 1 is a schematic representation of an ECMO system in accordance with an embodiment of the present disclosure;
FIG. 2 is a cross-sectional schematic representation of a fiber bundle of an oxygenator in accordance with an embodiment of the present disclosure;
FIG. 3 is an elevational view of an oxygenator and funnel capture system in accordance with an embodiment of the present disclosure;
FIG. 4 is a perspective view of an oxygenator and funnel capture system in accordance with an embodiment of the present disclosure; and
FIG. 5 is a schematic representation of an ECMO system in accordance with another embodiment of the present disclosure.

### DETAILED DESCRIPTION

Nasal cannulation is often used to provide supplemental oxygen as patients begin to feel better and are encouraged to ambulate to enhance their recovery. A patient's oxygen requirements increase as the patient ambulates and using nasal cannulation is quite beneficial to enable more effective ambulation and as the patient is being weaned from ECMO therapy. Imparting oxygen via nasal cannulation can complicate the patient's treatment because nasal cannulation has required its own oxygen source in addition to the oxygen source employed by the ECMO. The patient might thus be burdened with additional equipment, and this may occur as the patient is beginning to ambulate. This all occurs at a critical time in the recovery process because the ECMO therapy cannot be discontinued at this early stage. A weaning trial is typically implemented whereby the blood flow through the ECMO is steadily decreased while the patient's response to the decrease in reliance on the ECMO is closely monitored.

A patient's ambulation is often cumbersome due in part to the requirement for an oxygen source for the nasal cannulation. As a result, the patient is saddled with considerable equipment as the patient moves about. This includes the oxygenator system components and the oxygen source for the nasal cannulation in addition to any drip medications or other systems critical to the care of the patient. As ambulation is itself critical to recovery and mental well-being, it is desirable to unburden the patient from medical baggage to enable the patient to move about more freely and comfortably.

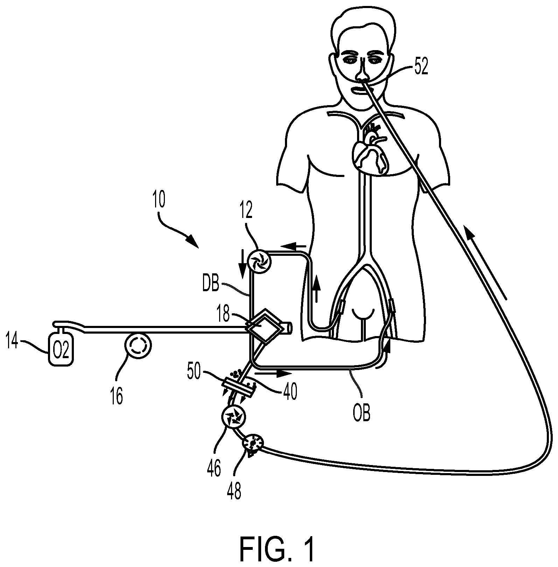

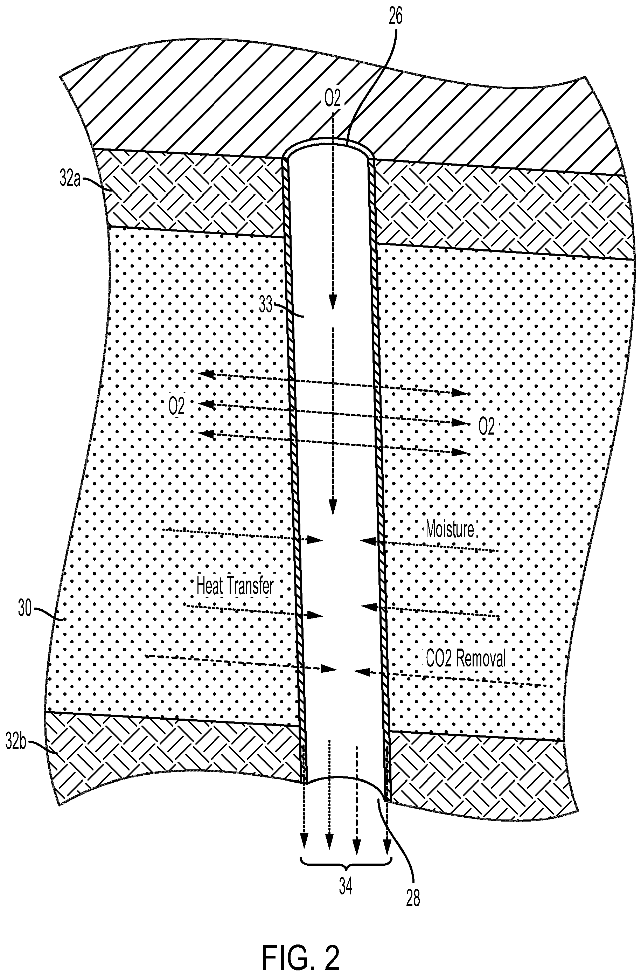

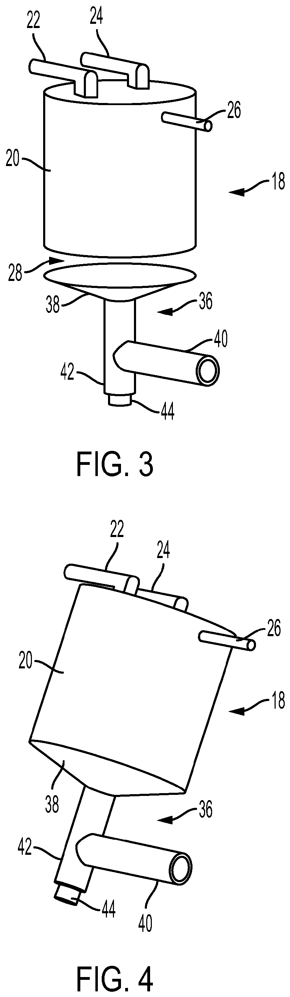

Referring to FIG. 1, there is illustrated an ECMO system generally designated as 10 and including a blood pump 12, an oxygen source 14, an oxygen pump 16 and an oxygenator 18. Shown more specifically in FIG. 3, the oxygenator 18 includes a housing 20, a blood inlet 22, a blood outlet 24, an oxygen inlet 26 and a gas discharge outlet 28. A hollow fiber bundle 30, as shown in FIG.2, made of semi-permeable hollow fibers, is encased by an upper cap 32a and a lower cap 32b within the housing 20 to secure the hollow fibers in the housing. A gas conduit 33 extends between the oxygen inlet 26 and gas discharge outlet 28.

The present disclosure contemplates any suitable arrangement of an oxygenator. By way of example, any suitable fiber bundle may be used and/or the inlets and outlets can be oriented in any suitable arrangement.

In use, deoxygenated blood DB is pumped to the blood inlet 22 of the oxygenator 18, while the oxygen is pumped to the oxygen inlet 26 of the oxygenator 18. Oxygenated blood OB exits the oxygenator 18 at the blood outlet 24 and is circulated back into the patient. Optionally, a blood warming device may be used to warm the blood being circulated back into the patient.

As shown in FIG. 2, pressurized oxygen enters the fiber bundle 30 and is infused into the blood, while carbon dioxide is forced from the blood into the conduit 33 along with excess oxygen. In addition, the blood also imparts moisture and warmth into the conduit 33. The gas discharge, designated as 34 in FIG. 2, is thus a mixture of warm and moist oxygen and carbon dioxide. The gas discharge 34 can be more than 85% oxygen which is far greater than the approximately 21% oxygen present in air. Typically, such gas discharge is expelled into the environment, at times with a sponge lapping up excess moisture.

Oxygen from oxygen sources in hospitals is dry and cold. Such oxygen for nasal cannulation can cause dryness in the upper airway mucosa of a patient, and thus add another level of discomfort for the patient. This can lead to nasal airway resistance and may also cause issues with intubation should intubation be required. Yet, the oxygen-rich gas discharge 34 from the ECMO, being moist and warm, can avoid discomfort and enhance comfort, as well as provide other medical benefits to a patient. The oxygen-rich gas discharge 34 can thus be captured and becomes supplemental oxygen for use in nasal cannulation or other uses where supplemental oxygen is beneficial while a patient is still on ECMO.

FIGS. 3 and 4 illustrate an embodiment of a collection device for capturing the gas discharge 34 from the oxygenator 18. Thus, a collection structure 36 can be positioned at the gas discharge outlet 28, either adjacently or in contact with it. The collection structure 36 includes a funnel portion 38, a cannula 40 and a moisture trap 42 for collecting excess moisture in the gas discharge 34. The moisture trap 42 can capture excess water vapor to prevent accumulation in the system. A sponge or removeable plug, both designated as 44 in FIGS. 3 and 4, can be provided. The sponge 44 can be removed and replaced from time to time, or the plug 44 can be removed for access to the trap and conduit of the collection structure 36. If desirable, the moisture content in the collected supplemental oxygen can be regulated in any suitable manner such as removing moisture to arrive at a therapeutically desirable level.

The cannula conduit 40 carries the supplemental oxygen to the nasal cannula nosepiece 52 (shown in FIG. 1). While the supplemental oxygen can be carried through the cannula conduit 40 should it remain sufficiently pressurized from the oxygen pump 16, a supplemental oxygen pump 46 can be employed. The supplemental oxygen can also be metered as desired with a flowmeter or gas regulator 48, shown schematically adjacent the supplemental oxygen pump 46, but can be arranged in any suitable location or as part of the pump 46. Also shown schematically is an air filter 50 which may be used to filter the supplemental oxygen for nasal cannulation. Such regulators and filters are known, and any suitable systems may be used.

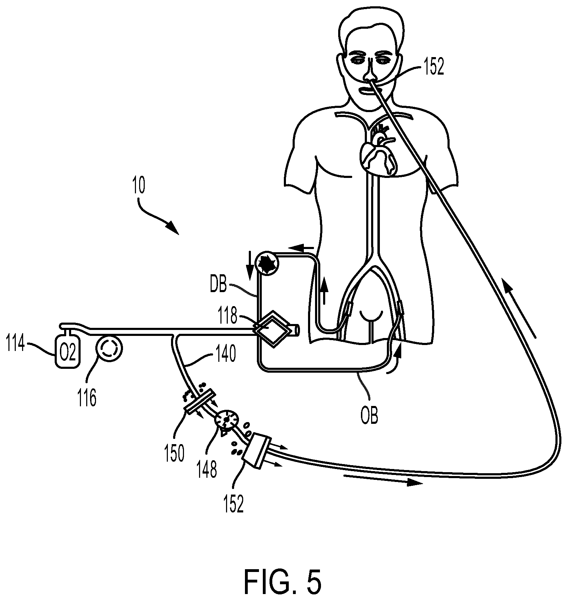

FIG. 5 illustrates schematically another embodiment of an ECMO system generally designated as 110 and by which supplemental oxygen is directed to a nasal cannula nosepiece 152. In this embodiment, an oxygenator 118 receives oxygen from the oxygen source 114 via pump 116. A portion of the oxygen from the oxygen source 114 is diverted as supplemental oxygen into conduit 140 where it is directed to nasal cannula nosepiece 152. A supplement pump may not be necessary but may be used. A filter 150 and a flowmeter or gas regulator 148 may be desirable. As the oxygen will not be warm and moist as in the other embodiment, a humidifier 152, may be provided. Oxygen warming and/or humidifying systems are known, such as that used in the heated and humidified high flow therapy nasal cannula system offered by Vapotherm, Inc. of Exeter, New Hampshire and also as described in U.S. Patent No. 11,103,670. Any suitable humidifying system may be used.

The present disclosure contemplates any suitable positioning of the various optional components, such as the air filter, warming system, humidifying system and pumps. Some preferences for embodiments may include having the oxygen pump for assistive pumping after capturing the supplemental oxygen located downstream from an air filter.

Although the present invention has been described with reference to particular embodiments, these embodiments are merely illustrative. There are many embodiments that can be developed and are encouraged to be developed yet remain within the spirit and scope of the claims which follow.

## Claims

1. A blood oxygenator, comprising:
a housing having an oxygenator fiber bundle, a blood inlet to the bundle, a blood outlet, an oxygen inlet to the bundle for oxygenating the blood of a patient and an oxygen outlet;
an oxygen source to provide oxygen to the oxygen inlet;
a pump for pumping oxygen through an oxygen feed conduit to the fiber bundle;
a supplemental oxygen device to administer oxygen to the patient separately from the oxygenation of the blood; and
wherein a portion of oxygen from the oxygen source is provided to the supplemental oxygen device.

2. The blood oxygenator in claim 1, including a collection device to capture gas discharged from the oxygenator at the oxygen outlet to provide the oxygen to the supplemental oxygen device.

3. The blood oxygenator in any one of claims 1 or 2, wherein the supplemental oxygen device is a nasal cannula.

4. The blood oxygenator in any one of claims 1, 2 or 3, including an air filter located after the oxygen outlet.

5. The blood oxygenator in any one of claims 1 or 4, including an oxygen pump located after the oxygen outlet.

6. The blood oxygenator in claim 5, wherein the oxygen pump is located downstream from the air filter.

7. The blood oxygenator in any one of claims 1-6, including an oxygen pressure regulator after the oxygen outlet.

8. The blood oxygenator in claim 1, including a diverter device upstream of the oxygen inlet to provide the oxygen to the supplemental oxygen device.

9. The blood oxygenator in any one of claims 1 or 8, wherein the supplemental oxygen device is a nasal cannula.

10. The blood oxygenator in any one of claims 1, 7 or 8, including an air filter located after the oxygen outlet.

11. The blood oxygenator in any one of claims 1, 7-9, including a pump located after the oxygen outlet.

12. The blood oxygenator in any one of claims 10 or 11, including a humidifying device located after the oxygen outlet.

13. The blood oxygenator in any one of claims 10 or 11, including a warming and humidifying device located after the oxygen outlet.

14. A method of administering oxygen to a patient, comprising the steps of:
providing a housing having within it a fiber bundle and a blood inlet, a blood outlet, an oxygen inlet and an oxygen outlet;
directing blood from a patient through the blood inlet into the fiber bundle;
pumping oxygen through an oxygen feed conduit extending from an oxygen source to the oxygen inlet, whereby an amount of oxygen is imparted into the fiber bundle to infuse the blood with oxygen; and
directing a portion of the oxygen pumped to the oxygen inlet for supplemental oxygen administration to the patient separately from the oxygenation of the blood.

15. An oxygenation system comprising:
a blood oxygenator having a housing with an interior, an oxygenator fiber bundle in the interior, a blood inlet to the bundle, a blood outlet, an oxygen inlet to the bundle for oxygenating the blood of the patient and an oxygen outlet;
an oxygen source to provide oxygen to the oxygen inlet;
a pump for pumping oxygen through an oxygen feed conduit to the fiber bundle;
a supplemental oxygen device to administer oxygen to the patient separately from the oxygenation of the blood; and
a collection device to capture gas discharged from the oxygenator at the oxygen outlet to provide the oxygen to the supplemental oxygen device.
